(19) Europäisches Patentamt
European Patent Office
Office européen des brevets

(11) Publication number: 0 113 534
A1

# (12) EUROPEAN PATENT APPLICATION

(21) Application number: 83307350.5

(22) Date of filing: 02.12.83

(51) Int. Cl.[3]: C 07 D 307/83
A 61 K 31/34

(30) Priority: 04.12.82 GB 8234670
04.12.82 GB 8234671

(43) Date of publication of application: 18.07.84 Bulletin 84/29

(84) Designated Contracting States: BE CH DE FR GB IT LI LU NL SE

(71) Applicant: Lilly Industries Limited
Lilly House Hanover Square
London W1R 0PA(GB)

(72) Inventor: Baker, Stephen Richard
Orchard Close Oaklea Drive
Eversley Hampshire(GB)

(72) Inventor: Jamieson, William Boffey
"Inverkip" 31, Kettlewell Close
Horsell Woking Surrey(GB)

(72) Inventor: Lindstrom, Terry Donald
1335 West 79th Street
Indianapolis Indiana 46260(US)

(72) Inventor: Ross, William James
8, Keswick Drive
Lightwater Surrey(GB)

(74) Representative: Hudson, Christopher Mark et al,
European Patent Attorney Erl Wood Manor
Windlesham Surrey GU20 6PH(GB)

(54) **Pharmaceutically active benzofuranone compounds.**

(57) Compounds of the following formula are disclosed

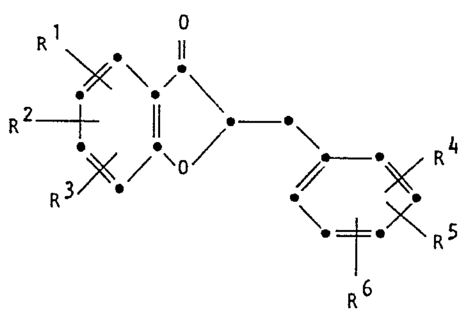

(I)

in which $R^1$, $R^2$, $R^3$, $R^4$, $R^5$ and $R^6$ are the same or different and are hydrogen, halogen, $C_{1-4}$ alkyl, $C_{1-4}$ alkoxy, $C_{3-8}$ cycloalkyl, optionally substituted phenyl, $C_{1-4}$ haloalkyl, $C_{1-4}$ acylamino, $C_{1-4}$ alkylaminocarbonyl, amino, cyano, hydroxy, nitro, $C_{3-4}$ alkenyl, $C_{3-4}$ alkenyloxy, carboxy $C_{1-2}$ alkoxy, carboxyl, tetrazol-5-yl or carboxyvinyl, or $R^1$ and $R^2$ taken together at adjacent carbon atoms represent a phenyl ring; or a salt or ester thereof. The compounds have pharmaceutical activity.

EP 0 113 534 A1

Croydon Printing Company Ltd

NOVEL PHARMACEUTICAL COMPOUNDS AND THEIR PREPARATION

This invention relates to novel compounds, to a process for their production and to their use as pharmaceuticals.

British Patent 2 030 142 discloses some aurone compounds having pharmaceutical properties and of the formula

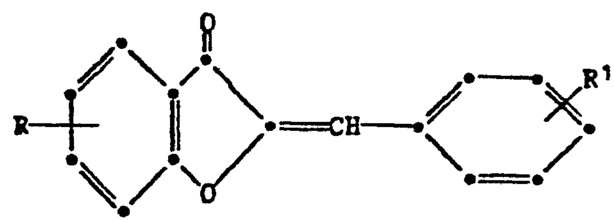

where R and $R^1$ take various substituent values. We have now discovered a group of related compounds which also possess useful pharmacological activity and that can be derived from them.

The compounds of the invention have the formula

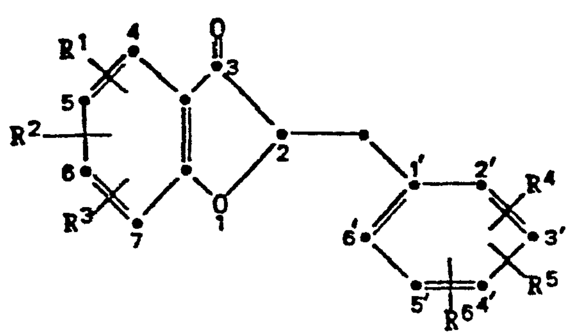

(I)

in which $R^1$, $R^2$, $R^3$, $R^4$, $R^5$ and $R^6$ are the same or different and are hydrogen, halogen, $C_{1-4}$alkyl, $C_{1-4}$alkoxy, $C_{3-8}$cycloalkyl, optionally substituted phenyl, $C_{1-4}$haloalkyl, $C_{1-4}$acylamino,

$C_{1-4}$alkylaminocarbonyl, amino, cyano, hydroxy, nitro, $C_{3-4}$alkenyl, $C_{3-4}$alkenyloxy, carboxy $C_{1-2}$ alkoxy, carboxyl, tetrazol-5-yl or carboxyvinyl or in which $R^1$ and $R^2$ taken together at adjacent carbon atoms represent a phenyl ring; or a salt or ester thereof.

A preferred group of compounds of the invention have the formula

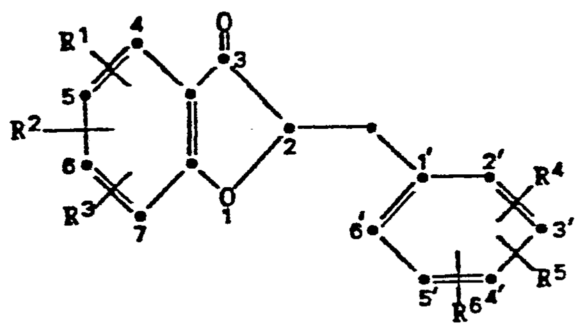

(I)

in which $R^1$, $R^2$ and $R^3$ are the same or different and are hydrogen, halogen, $C_{1-4}$alkyl, $C_{1-4}$alkoxy, $C_{3-8}$cycloalkyl, optionally substituted phenyl, $C_{1-4}$haloalkyl, $C_{1-4}$acylamino, $C_{1-4}$alkylaminocarbonyl, amino, cyano, hydroxy, nitro, $C_{3-4}$alkenyl, tetrazol-5-yl or carboxyvinyl or in which $R^1$ and $R^2$ taken together at adjacent carbon atoms represent a phenyl ring, at least one of $R^1$, $R^2$ and $R^3$ being other than hydrogen, and in which $R^4$, $R^5$, $R^6$ are each hydrogen, halogen, $C_{1-4}$alkyl, $C_{1-4}$alkoxy, $C_{3-8}$cycloalkyl, optionally substituted phenyl, $C_{1-4}$haloalkyl, $C_{1-4}$acylamino, $C_{1-4}$alkylaminocarbonyl, amino, cyano, hydroxy, nitro, $C_{3-4}$alkenyl, carboxyl, tetrazol-5-yl or carboxyvinyl, provided that at least one of $R^4$, $R^5$ and $R^6$ is carboxyl or at least one of $R^1$, $R^2$, $R^3$, $R^4$, $R^5$ and $R^6$ is tetrazol-5-yl or carboxyvinyl; or a salt or ester thereof.

A further preferred group of compounds of the invention have the formula

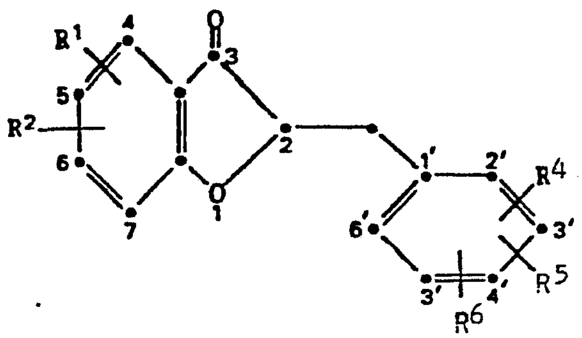

(II)

in which either $R^1$ and $R^2$ are both hydrogen or $R^1$ is carboxyl and $R^2$ is hydrogen, $C_{3-4}$alkenyl or $C_{1-4}$alkyl, and $R^4$, $R^5$ and $R^6$ are each hydrogen, nitro, hydroxyl, carboxyl, carboxy-$C_{1-2}$alkoxy, $C_{3-4}$alkenyl, $C_{1-4}$alkyl, $C_{3-4}$alkenyloxy, $C_{1-4}$alkoxy or the group

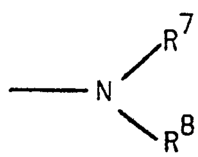

where $R^7$ and $R^8$ are each hydrogen or $C_{1-4}$alkyl; and salts and esters thereof.

The term "halogen" means especially chlorine, bromine and fluorine. The term "$C_{1-4}$alkyl" includes, for example, methyl, ethyl, propyl, isopropyl, butyl, tert butyl, being preferably methyl, ethyl or tert-butyl. The term "$C_{1-4}$alkoxy" includes, for example, methoxy, ethoxy, propoxy and butoxy, and is preferably methoxy. The term "$C_{3-8}$cycloalkyl" includes, for example, cyclopropyl, cyclopentyl and cycloheptyl, and is preferably cyclohexyl. The term "optionally substituted phenyl" includes, for example, phenyl optionally substituted with 1 to 3 substituents selected from methyl, methoxy, halogen

and nitro. The term "$C_{1-4}$haloalkyl" can be, for example, any of the groups listed for "$C_{1-4}$alkyl" substituted with one to three halo atoms such as fluorine or chlorine, and is especially trifluoromethyl. The term "$C_{3-4}$alkenyl" is preferably allyl. The term "$C_{1-4}$acylamino" includes, for example, acetamido and groups of the formula RCONH- where R has the value of $C_{1-3}$alkyl, and $C_{1-4}$alkylaminocarbonyl includes, for example, N-isopropylcarboxamido, and groups of the formula

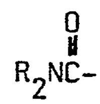

where R is hydrogen or $C_{1-4}$alkyl. "Amino" includes, for example, $-NH_2$, mono $C_{1-4}$alkylamino and di $C_{1-4}$alkylamino, especially dimethylamino and for example can be of the form

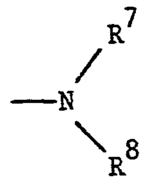

where $R^7$ and $R^8$ are each hydrogen or $C_{1-4}$alkyl. The term carboxy-$C_{1-2}$ alkoxy covers carboxymethoxy, 2-carboxyethoxy and 1-carboxyethoxy. The preferred substituents for $R^1$, $R^2$, $R^3$, $R^4$, $R^5$ and $R^6$ are hydrogen, halogen, $C_{1-4}$alkyl, $C_{1-4}$alkoxy, cyclohexyl, trifluoromethyl, dimethylamino, hydroxy, carboxyl, tetrazol-5-yl or carboxyvinyl.

A preferred group of compounds of formula (I) is one in which $R^1$, $R^2$ and $R^3$ are selected from hydrogen, halogen, $C_{1-4}$alkyl, $C_{1-4}$alkoxy, cyclohexyl, trifluoromethyl, dimethylamino, hydroxy, tetrazol-5-yl or carboxyvinyl at least one of $R^1$, $R^2$ and $R^3$ being other than hydrogen, in which $R^4$ is carboxyl and in which $R^5$ and $R^6$ are selected from hydrogen, halogen, $C_{1-4}$alkyl,

$C_{1-4}$alkoxy, cyclohexyl, trifluoromethyl, dimethylamino, hydroxy, carboxyl, tetrazol-5-yl or carboxyvinyl.

It is further preferred that $R^1$ be selected from $C_{1-4}$alkoxy, $C_{1-4}$alkyl and halogen and $R^2$ and $R^3$ are both hydrogen. Similarly $R^4$ is preferably chosen from carboxyl, tetrazol-5-yl or carboxyvinyl, and $R^5$ and $R^6$ are both hydrogen, and an especially preferred group of compounds are those having the following formula

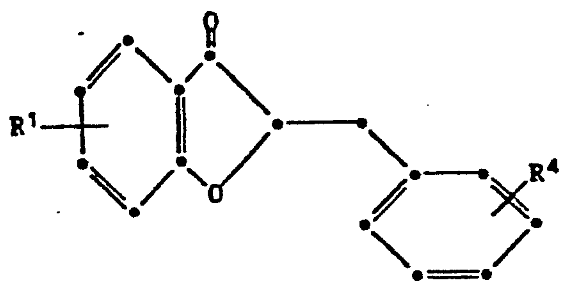

in which $R^1$ is $C_{1-4}$alkoxy, $C_{1-4}$alkyl or halogen and $R^4$ is carboxyl, tetrazol-5-yl or carboxyvinyl, or a salt or ester thereof.

A further preferred group of compounds is one of formula (II) above, in which $R^1$ is carboxyl (especially in the 5-position), $R^2$ (especially in the 7-position) is hydrogen, $C_{3-4}$alkenyl or $C_{1-4}$alkyl, and $R^4$, $R^5$ and $R^6$ are each hydrogen, hydroxyl, $C_{3-4}$alkenyl, $C_{1-4}$alkyl, $C_{3-4}$alkenyloxy, $C_{1-4}$alkoxy or the group

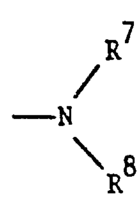

when $R^7$ and $R^8$ are each hydrogen or $C_{1-4}$alkyl and of those compounds the most preferred are those in which $R^1$ is carboxyl (especially those substituted in the 5-position), $R^2$ is hydrogen and $R^4$, $R^5$ and $R^6$ are each hydrogen, carboxyl, $C_{1-4}$alkyl or $C_{1-4}$alkoxy.

Suitable salts of compounds of invention include for example, those of mineral bases such as alkali metal hydroxides, especially the potassium or sodium salts, or alkaline earth metal hydroxides, especially the calcium salts, or of organic bases such as amines for example trimethylamine. When the compounds contain an amino group or a basic nitrogen atom, acid addition salts are included such as those with inorganic acids, for example hydrochloric, hydrobromic, nitric, sulphuric or phosphoric acids, or with organic acids, such as organic carboxylic acids, for example, glycollic, maleic, hydroxymaleic, fumaric, malic, tartaric, citric, salicylic, o-acetoxybenzoic, nicotinic or isonicotinic acid, or organic sulphonic acids for example methane sulphonic, ethane sulphonic, 2-hydroxyethane sulphonic, toluene-p-sulphonic or naphthalene-2-sulphonic acid. Preferred esters are those derived from $C_{1-4}$alkanols, for example the methyl, ethyl, propyl, isopropyl and n-butyl. Also included are esters having a substituted alkyl group in view of the fact that it is often desirable to attach an ester group that cleave to give the free acid, and examples of such substituted alkyls include acetoxymethyl, methylthiomethyl, methoxyethyl, ethoxyethyl, methylsulphinylmethyl and methylsulphonylmethyl.

The preferred salts and esters are those which are pharmaceutically-acceptable but other derivatives are also included in the invention since they may be useful as intermediates in the preparation, purification or characterisation of the pharmaceutical end product.

The invention includes a method of preparing compounds of the invention which comprises reducing a compound of formula

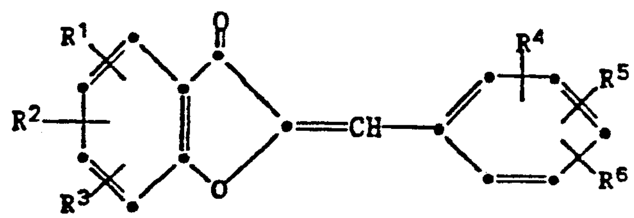

(III)

The reduction is preferably carried out by the use of hydrogen and a metal catalyst, the latter preferably being a precious metal such as platinum or palladium. It has been found that 10 per cent palladium on charcoal is a convenient catalyst for this purpose. Generally the reduction process is carried out in a solvent, for example, acetic acid, and at a temperature from 0°C to 100°C, for example at room temperature.

As mentioned above, the compounds of formula (III) are known and their method of preparation is described, for example, in British Patents 2 030 142, 2 014 566 and 2 001 631. They may best be prepared by condensing an appropriately substituted benzaldehyde with a benzofuranone as depicted below:

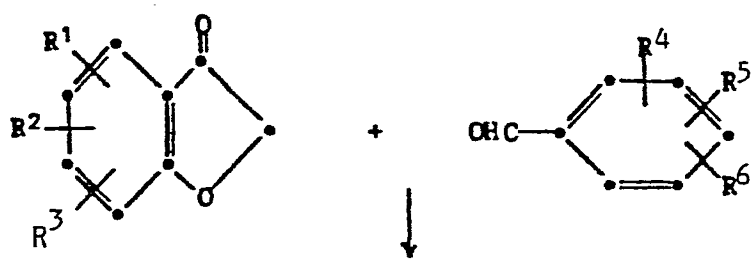

compound of formula (III)

It will be appreciated that many of the compounds of the invention can be converted one to another by introduction or conversion of groups in the benzofuranone or phenyl ring either during one of the intermediate stages in the preparative reactions or in an end product. Thus, for example, a tetrazol-5-yl compound can be prepared from the corresponding cyano compound by reaction with an azide. When a nitro substituent is desired in the benzofuranone or phenyl nuclei, the corresponding unsubstituted compound can be nitrated with a mixture of concentrated nitric and sulphuric acids by the conventional method. The nitro compound can subsequently be converted to other substituents such as amino or acylamino. The amino compound may be diazotised and the resultant diazonium salt converted to a variety of other products, for example, by decomposition in an alcohol to yield the corresponding alkoxy substituted compound or by reaction with a cuprous halide to yield the corresponding halo substituted compound. Hydroxy substituted compounds can be prepared from the corresponding methoxy compounds by cleavage with, for example, boron tribromide.

It will be appreciated that the compounds of the invention have an asymmetric centre at the carbon atom of the benzofuranone moiety linked to the benzyl group (carbon number 2) and this asymmetry gives rise to the existence of optical isomers. Substantially pure optical isomer can be prepared by chemical reaction in the presence of a chiral catalyst. Alternatively, a racemic mixture of both isomers can be separated by conventional chemical methods such as for example by the preparation of diastereoisomers as salts with an optically-active base and subsequent liberation of the enantiomers.

The anti-allergy activity of the compounds of the invention and their pharmaceutically acceptable salts and esters has been demonstrated in guinea pigs using either the "guinea-pig chopped lung test" described by Mongar and Schild in the Journal of Physiology (London) 131, 107(1956) or Brocklehurst in the Journal of Physiology (London) 151, 416 (1960), or the "Herxheimer" test described in the Journal of Physiology (London) 117, 251(1952). In the "Herxheimer" test, which is based on an allergic bronchospasm induced in guinea pigs closely resembling an asthmatic attack in man, compounds exhibited activity at dosages ranging from 25 mg/kg to 200 mg/kg.

The compounds of the invention have also shown activity in tests devised to indicate anti-inflammatory activity as, for example, the adjuvant arthritis test (B.B. Newbould Chemotherapy of Arthritis Induced in Rats by Mycobacterial Adjuvant. Br. J. Pharmacol. 21, 127-136 (1963)).

The compounds of this invention are accordingly indicated for therapeutic use in the treatment of immediate sensitivity reactions and in particular in the treatment of asthma, as well as being of use in the treatment of antiinflammatory diseases.

The compounds of this invention may be administered by various routes, for example, by the oral or rectal route, by inhalation, topically or parenterally, for example by injection, being usually employed in the form of a pharmaceutical composition. Thus the invention includes a method of administering a compound according to formula I to a mammal suffering from an allergic reaction or an inflammatory disease or to prevent such allergic or inflammatory disease. Pharmaceutical compositions also form part of the present invention and are prepared in a manner well known in the pharmaceutical art and normally comprise at leat one active compound in association with a pharmaceutically acceptable diluent or carrier. In making the compositions of the present invention, the active ingredient will usually be mixed with a carrier, or diluted by a carrièr, and/or enclosed within a carrier which may, for example, be in the form of a capsule, sachet, paper or other container. Where the carrier serves as a diluent, it may be a solid, semi-solid, or liquid material which acts as a vehicle, excipient or medium for the active ingredient. Thus, the composition may be in the form of tablets, lozenges, sachets, cachets, elixires, suspensions, aerosols as a solid or in a liquid medium, ointments containing for example up to 10% by weight of the active compound, soft and hard gelatin capsules, suppositories, injection solutions

and suspensions and sterile packaged powders. For administration by inhalation, particular forms of presentation include aerosols, atomisers and vaporisers.

Some examples of suitable carriers are lactose, dextrose, sucrose, sorbitol, mannitol, starches, gum acacia, calcium phosphate, alginates, tragacanth, gelatin, syrup, methyl cellulose, methyl- and propyl- hydroxybenzoate, talc, magnesium stearate and mineral oil. The compositions of the invention may, as is well known in the art, be formulated so as to provide quick, sustained or delayed release of the active ingredient after administration to the patient.

Where the compositions are formulated in unit dosage form, it is preferred that each unit dosage form contains from 5 mg to 500 g, more usually 25 to 200 mg, of the active ingredient. The term "unit dosage form" refers to physically discrete units suitable as unit dosages for human subjects and animals, each unit containing a predetermined quantity of active material calculated to produce the desired therapeutic effect, in association with the required pharmaceutical carrier.

The active compounds are effective over a wide dosage range and, for example, dosages per day will normally fall withing the range of from 0.5 to 300 mg/kg, more usually in the range of from 5 to 100 mg/kg. However, it will be understood that the amount administered will be determined by the physician in the light of the relevant circumstances including the condition to be treated, the choice of compound to be administered and the chosen route of administration, and therefore

the above dosage ranges are not intended to limit the scope of the invention in any way.

The following Examples illustrates the invention.

EXAMPLES 1 to 3

3-[(2,3-Dihydro-5-methoxy-3-oxo-2-benzofuranyl)methyl]benzoic acid

(Z)-3-[(5-Methoxy-3-oxo-2-(3H)-benzofuranylidene)methyl] benzoic acid (10 g) was suspended in glacial acetic acid (250 ml) and hydrogenated at room temperature and 60 p.s.i. pressure in the presence of 10% palladium on charcoal (500 mg) for 1 hour. The catalyst was filtered off and the nearly colourless filtrate evaporated in vacuo to give a viscous oil which readily crystallised. This crystalline solid was slurried with a little diethyl ether and refiltered to give the title compound as an off-white crystalline solid, m.p. 143-152°C (with decomposition).

The following compounds were prepared in a similar manner:

4-[(2,3-Dihydro-5-methoxy-3-oxo-2-benzofuranyl)methyl]benzoic acid, m.p. 168°C (with decomposition)

4-[(2,3-Dihydro-6-methyl-3-oxo-2-benzofuranyl)methyl]benzoic acid, m.p. 188-190°C (with decomposition).

EXAMPLE 4

(a) Z-3-[(2,3-Dihydro-5-methoxycarbonyl-3-oxo-2-benzofuranyl)-methylene]benzoic acid

5-Methoxycarbonylbenzofuran-3-(2H)one (4.9 g) was dissolved in warm dioxan (50 ml). 3-Carboxybenzaldehyde (4.5 g) was then added followed by concentrated hydrochloric acid

(10 ml). The mixture was heated on a steam bath for 15 minutes with formation of yellow crystals. After cooling and addition of an equal volume of water, the crystalline product was filtered off and recrystallised from dimethylformamide to give the title compound, m.p. 280°C.

(b) 3-[(2,3-Dihydro-5-methoxycarbonyl-3-oxo-2-benzofuranyl) methyl]benzoic acid

The product from (a) (1 g) was suspended in glacial acetic acid (100 ml) and hydrogenated at 60 p.s.i. in the presence of 10% palladium on carbon (100 mg). After 1 hour the catalyst was filtered off and the colourless filtrate evaporated in vacuo to give a light straw coloured oil which crystallised on standing. Recrystallisation from ethyl acetate/petrol gave the required product as off-white crystals, m.p. 140-143°C.

CLAIMS

1. A compound of the formula

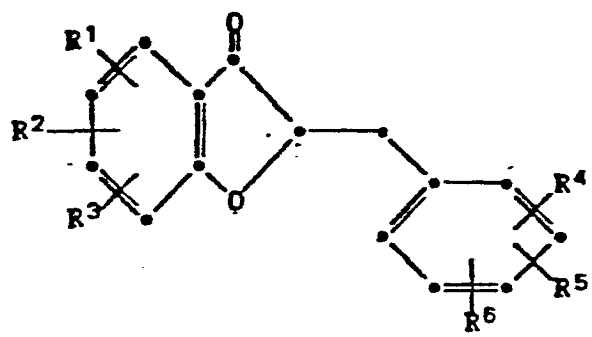

(I)

in which $R^1$, $R^2$, $R^3$, $R^4$, $R^5$ and $R^6$ are the same or different and are hydrogen, halogen, $C_{1-4}$ alkyl, $C_{1-4}$ alkoxy, $C_{3-8}$ cycloalkyl, optionally substituted phenyl, $C_{1-4}$ haloalkyl, $C_{1-4}$ acylamino, $C_{1-4}$ alkylaminocarbonyl, amino, cyano, hydroxy, nitro, $C_{3-4}$ alkenyl, $C_{3-4}$ alkenyloxy, carboxy $C_{1-2}$ alkoxy, carboxyl, tetrazol-5-yl or carboxyvinyl, or $R^1$ and $R^2$ taken together at adjacent carbon atoms represent a phenyl ring; or a salt or ester thereof.

2. A compound according to claim 1 in which $R^1$, $R^2$ and $R^3$ are the same or different and are hydrogen, halogen, $C_{1-4}$ alkyl, $C_{1-4}$ alkoxy, $C_{3-8}$ cycloalkyl, optionally substituted phenyl, $C_{1-4}$haloalkyl, $C_{1-4}$acylamino, $C_{1-4}$alkylaminocarbonyl, amino, cyano, hydroxy, nitro, $C_{3-4}$alkenyl, tetrazol-5-yl or carboxyvinyl or in which $R^1$ and $R^2$ taken together at adjacent carbon atoms represent a phenyl ring, at least one of $R^1$, $R^2$ and $R^3$ being other than hydrogen, and in which $R^4$, $R^5$, $R^6$ are

each hydrogen, halogen, $C_{1-4}$alkyl, $C_{1-4}$alkoxy, $C_{3-8}$cycloalkyl, optionally substituted phenyl, $C_{1-4}$haloalkyl, $C_{1-4}$acylamino, $C_{1-4}$alkylaminocarbonyl, amino, cyano, hydroxy, nitro, $C_{3-4}$alkenyl, carboxyl, tetrazol-5-yl or carboxyvinyl, provided that at least one of $R^4$, $R^5$ and $R^6$ is carboxyl or at least one of $R^1$, $R^2$, $R^3$, $R^4$, $R^5$ and $R^6$ is tetrazol-5-yl or carboxyvinyl; or a salt or ester thereof.

3. A compound according to claim 2 in which $R^1$, $R^2$ and $R^3$ are selected from hydrogen, halogen, $C_{1-4}$alkyl, $C_{1-4}$alkoxy, cyclohexyl, trifluoromethyl, dimethylamino, hydroxy, tetrazol-5-yl or carboxyvinyl at least one of $R^1$, $R^2$ and $R^3$ being other than hydrogen, in which $R^4$ is carboxyl and in which $R^5$ and $R^6$ are selected from hydrogen, halogen, $C_{1-4}$alkyl, $C_{1-4}$alkoxy, cyclohexyl, trifluoromethyl, dimethylamino, hydroxy, carboxyl, tetrazol-5-yl or carboxyvinyl.

4. A compound according to claim 2 in which $R^1$ is $C_{1-4}$alkoxy, $C_{1-4}$alkyl or halogen and $R^2$ and $R^3$ are both hydrogen, $R^4$ is carboxyl, tetrazol-5-yl or carboxyvinyl, and $R^5$ and $R^6$ are both hydrogen.

5. A compound according to claim 1 of the formula (II)

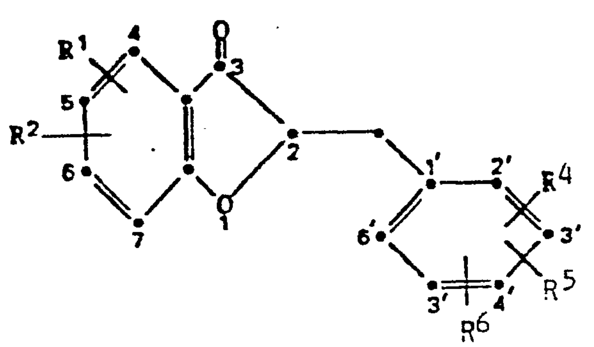

(II)

in which either $R^1$ and $R^2$ are both hydrogen or $R^1$ is carboxyl and $R^2$ is hydrogen, $C_{3-4}$alkenyl or $C_{1-4}$alkyl, and $R^4$, $R^5$ and $R^6$

are are each hydrogen, nitro, hydroxyl, carboxyl, carboxy-$C_{1-2}$alkoxy, $C_{3-4}$alkenyl, $C_{1-4}$alkyl, $C_{3-4}$alkenyloxy, $C_{1-4}$alkoxy or the group

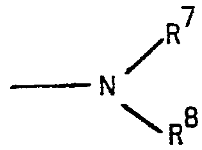

where $R^7$ and $R^8$ are each hydrogen or $C_{1-4}$alkyl; and salts and esters thereof.

6. A compound according to claim 5 in which $R^1$ is carboxyl $R^2$ is hydrogen, $C_{3-4}$alkenyl or $C_{1-4}$alkyl, and $R^3$, $R^4$ and $R^5$ are each hydrogen, hydroxyl, $C_{3-4}$alkenyl, $C_{1-4}$alkyl, $C_{3-4}$alkenyloxy, $C_{1-4}$alkoxy or the group

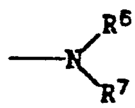

when $R^6$ and $R^7$ are each hydrogen or $C_{1-4}$alkyl.

7. A pharmaceutical formulation comprising a compound according to claim 1 or a pharmaceutically acceptable salt thereof, admixed with a diluent or carrier therefor.

8. A process for producing a compound according to claim 1 which comprises reducing a compound of formula

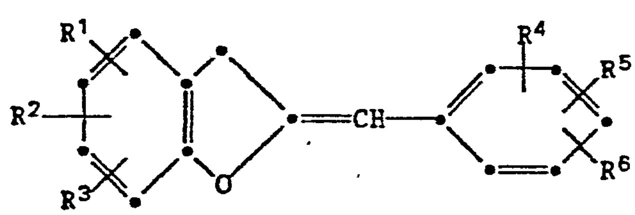

in which $R^1$, $R^2$, $R^3$, $R^4$, $R^5$ and $R^6$ have the values defined in claim 1.

9. A compound according to claim 1 or a pharmaceutically-acceptable salt thereof, for use as a pharmaceutical.

European Patent Office

# EUROPEAN SEARCH REPORT

0113534
Application number

EP 83 30 7350

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | JOURNAL OF THE CHEMICAL SOCIETY, PERKIN TRANSACTIONS 1, 1981 (LONDON, GB) B.C.B. BEZUIDENHOUDT et al. "A novel alpha-hydroxydihydrochalcone from the heartwood of ptecocarpus angolensis D.C.: Absolute configuration, synthesis, photochemical transformations, and conversion into alpha-methyldeoxybenzoins", pages 263-269 * page 265, formula 13 * --- | 1-9 | C 07 D 307/83<br>A 61 K 31/34 |
| X,Y | JOURNAL OF THE CHEMICAL SOCIETY, CHEMICAL COMMUNICATIONS, no. 18, September 17, 1975 (LONDON, GB) M.F. GRUNDON et al. "Oxidative cyclisation of 2'-hydroxychalcones to aurones using mercury (II) acetate in dimethyl sulphoxide", pages 772,773 * page 773, formula 3 * --- | 1-9 | TECHNICAL FIELDS SEARCHED (Int. Cl. 3)<br>C 07 D 307/00 |
| X,Y | JOURNAL OF THE CHEMICAL SOCIETY, PERKIN TRANSACTIONS I, 1972 (LONDON, GB) J.S. HASTINGS et al. "The stereochemistry of aurones [2-substituted benzylidenebenzofuran-3(2H)-ones] ", pages 2128-2132 * the entire document * --- -/- | 1-9 | |

The present search report has been drawn up for all claims

| Place of search | Date of completion of the search | Examiner |
|---|---|---|
| THE HAGUE | 28-02-1984 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0113534

Application number

EP 83 30 7350



## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
| --- | --- | --- | --- |
| X,Y | JOURNAL OF THE CHEMICAL SOCIETY, 1960, (LONDON, GB) A.J. BIRCH et al. "The structure of alphitonin", pages 3593-3599 * page 3594, formula II * --- | 1-9 | |
| X,Y | JOURNAL OF THE AMERICAN CHEMICAL SOCIETY, volume 60, no. 4, April 8, 1938 (COLUMBUS, OHIO, US) R.L. SHRINER et al. "Derivatives of coumaran. I. 2-benzyl-5(and 6 )-methoxycoumaran-3-one", pages 894-896 * the whole article * --- | 1-9 | |
| X,Y | US-A-3 627 763 (K.A. JAEGGI) * the whole document, particularly column 2, formula IV * --- | 1-9 | TECHNICAL FIELDS SEARCHED (Int. Cl. ³) |
| D,Y | GB-A-2 030 142 (LILLY IND.) * the whole document * ----- | 1-9 | |

The present search report has been drawn up for all claims

| Place of search | Date of completion of the search | Examiner |
| --- | --- | --- |
| THE HAGUE | 28-02-1984 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82